(19) Europäisches Patentamt
European Patent Office
Office européen des brevets

(11) EP 2 062 601 A1

(12) EUROPEAN PATENT APPLICATION
published in accordance with Art. 153(4) EPC

(43) Date of publication:
27.05.2009 Bulletin 2009/22

(21) Application number: 07807047.1

(22) Date of filing: 11.09.2007

(51) Int Cl.:
*A61L 15/00* (2006.01) *A61L 31/00* (2006.01)
*C08J 9/02* (2006.01) *C08J 9/04* (2006.01)

(86) International application number:
PCT/JP2007/067638

(87) International publication number:
WO 2008/032697 (20.03.2008 Gazette 2008/12)

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(30) Priority: 11.09.2006 JP 2006245567
17.10.2006 JP 2006282828

(71) Applicant: Kaneka Corporation
Kita-ku
Osaka-shi, Osaka 530-8288 (JP)

(72) Inventors:
- GOTO, Masaoki
Settsu-shi
Osaka 566-0072 (JP)
- MATSUMURA, Yoichi
Settsu-shi
Osaka 566-0072 (JP)
- TANIGUCHI, Shuhei
Settsu-shi
Osaka 566-0072 (JP)
- SHIBAYA, Miaki
Settsu-shi
Osaka 566-0072 (JP)
- FUKAYA, Kohei
Settsu-shi
Osaka 566-0072 (JP)
- SANEYASU, Takaoki
Settsu-shi
Osaka 566-0072 (JP)
- ICHIMURA, Masaki
Settsu-shi
Osaka 566-0072 (JP)

(74) Representative: Vossius & Partner
Siebertstrasse 4
81675 München (DE)

# (54) FOAM AND PROCESS FOR PRODUCING THE SAME

(57) It is intended to provide a resin foam which can be preferably used for medical use, and a flexible silicone type resin foam having a water-absorbing property. The resin foam which has a low toxicity and excellent physical properties for medical use such as moisture permeability, skin compatibility and low irritation can be obtained by using a resin which has a siloxane unit and an oxyalkylene unit in its molecular structure and does not contain a unit derived from an isocyanate group. Further, the flexible silicone type resin foam having a water-absorbing property can be obtained by allowing it to have a specific resin composition and a foam structure.



Printed by Jouve, 75001 PARIS (FR)

## Description

### Technical Field

The present invention relates to a foam and process for producing the same.

### Background Art

A foam containing bubbles in resin is excellent in a cushioning property, a heat insulating property, shock absorption, lightweightness, a water absorbing property, moisture permeability, etc., and is utilized in various fields, such as automotive parts such as a bumper and a seat cushion; building materials such as a heat insulating material and a panel; and foodstuff applications such as a fish box and a foodstuff wrapping material. Also for medical use, the foam is used in a care pad, a foot care pad, a catheter fixing sheet, a bleeding stop pad, a wound dressing material, and the like. As material resin of a resin foam used for the medical use, polyurethane, silicone resin, polyolefine, polystyrene, etc., are used.

For example, it is known that a wound dressing material which is effective particularly in healing a serious injury is obtained using a hydrophilic polyurethane resin foam (e.g., Patent Document 1). However, polyurethane has had concerns regarding odor of a remaining reacted catalyst or toxicity of unreacted isocyanate, and has sometimes applied uncomfortable feeling upon using due to swelling at the time of water absorption. Therefore, attempts to suppress swelling of a water absorbing foam have been made (Patent Document 2). However, preferably used is urethane and the problem with toxicity concerning has not yet been solved.

In contrast, a wound dressing material using a silicone resin foam is also known (e.g., Patent Document 3). By the use of silicone resin excellent in moisture permeability, flexibility, etc., a wound dressing material is obtained which is free from steaming, is soft on a wound surface, and has less concern regarding toxicity of isocyanate or the like. The silicone resin foam has problems that the compatibility with the skin is low, the water absorbing property tends to be low, and it is difficult to apply to a wound with high exudate.

Conventionally, the silicone foam is known to have excellent physical properties, such as heat resistance, hydrophobicity, low temperature resistance, weather resistance, electrical insulating property, and flexibility, compared with a foam containing another kind of high molecular weight substance, such as a urethane foam (e.g., Patent Document 4). However, since silicone generally has low surface tension, the silicone foam is difficult to obtain a water absorbing property. Thus, the silicone foam is difficult to use for fields requiring a water absorbing property, such as a makeup puff, a toiletry material, an agricultural material, a gardening flooring material, and a cleaning material.

In contrast, as a measure to improve coating and adhesion properties of the silicone foam, a foam is known which contains a silicone type resin composition containing an organic compound which has a carbon-carbon double bond; whose molecular structure is at least one selected from the group consisting of a polyether-type organic polymer skeleton, a phenolformaldehyde-type organic polymer skeleton, and a bisphenol A-type monomer skeleton; and which does not contain a siloxane unit in the molecular structure and chain polyorganohydrogensiloxane (e.g., Patent Document 5). However, the findings on the improvement in a water absorbing property are not seen.

Patent Document 1: Japanese Patent No. 3541948
Patent Document 2: Japanese Unexamined Patent Publication (Translation of PCT Application) No. 2005-516735
Patent Document 3: Japanese Examined Patent Publication No. 7-51139 Patent Document 4: Japanese Unexamined Patent Publication No. 9-124816 Patent Document 5: Japanese Patent No. 3569919

### Disclosure of the Invention

### Technical Problems to be Solved

Under the circumstances, an object of the present invention is to provide a resin foam which can be preferably used for medical use and a flexible silicone type resin foam having a water absorbing property.

### Means to Solve the Problems

The present inventors have conducted extensive studies for solving the problems, and found that a resin foam which has a low toxicity and excellent physical properties for medical use, such as moisture permeability, skin compatibility, and low irritation can be obtained by the use of resin containing a siloxane unit and an oxyalkylene unit in the molecular structure and containing no isocyanate derived unit and also found that a flexible silicone type resin foam having a water absorbing property can be obtained by allowing it to have a specific resin composition and a foam

structure. Thus, the present invention has been accomplished.

More specifically, the present invention relates to a resin foam for medical use containing resin containing a siloxane unit and an oxyalkylene unit in the molecular structure and containing no isocyanate derived unit.

The present invention also relates to a resin foam for medical use, in which the water absorption represented by equation (1) when immersed in a physiological sodium chloride solution at 37°C for 24 hours is 200 wt% or more and lower than 2000 wt%:

$$\text{Water absorption} = 100 \times (\text{Foam weight after immersion} - \text{Foam weight before immersion}) / (\text{Foam weight before immersion}) \quad (1).$$

The present invention also relates to a flexible water absorbing resin foam containing resin containing a siloxane unit and an oxyalkylene unit in the molecular structure and containing no isocyanate derived unit, in which the water absorption represented by equation (1) shown above when immersed in a physiological sodium chloride solution at 37°C for 24 hours is 200 wt% or more and lower than 2000 wt%.

The present invention also relates to a resin foam in which the resin contains an oxyethylene unit as at least one kind of the oxyalkylene unit.

The present invention also relates to a resin foam in which the resin contains an oxyethylene unit in a proportion of 5 wt% or more and lower than 80 wt%.

The present invention also relates to a resin foam in which the resin is obtained by curing a mixture containing:

an alkenyl group-containing compound (A);
a hydrosilyl group-containing compound (B); and
a hydrosilylation catalyst (C).

The present invention also relates to a resin foam in which the alkenyl group-containing compound (A) is an organic compound containing no siloxane unit in the molecular structure.

The present invention also relates to a resin foam in which the alkenyl group-containing compound (A) contains 50 wt% or more of a polyoxyalkylene polymer containing at least one alkenyl group at the terminal.

The present invention also relates to a resin foam which is obtained by further blending a foaming agent (D) in the resin, and then foaming the mixture simultaneously with curing.

The present invention also relates to a resin foam in which at least one kind of the foaming agent (D) is an active hydrogen group-containing compound.

The present invention also relates to a resin foam in which the active hydrogen group-containing compound is a compound having an OH group.

The present invention also relates to a resin foam in which the compound having an OH group is at least one member selected from the group consisting of water, alcohol, and polyether polyol.

The present invention also relates to a resin foam in which at least one kind of the compound having an OH group is polyethylene glycol.

The present invention also relates to a resin foam in which the amount of a hydrosilyl group in the compound (B) is 2 mol equivalent or more relative to an alkenyl group in the alkenyl group-containing compound (A).

The present invention also relates to a resin foam in which the density is 10 $kg/m^3$ or more and lower than 500 $kg/m^3$ and the open cell coefficient is 80% or more.

The present invention also relates to a resin foam in which the density is 10 $kg/m^3$ or more and lower than 500 $kg/m^3$, the thickness is 1 mm or more and lower than 100 mm, and the open cell coefficient is 80% or more.

The present invention also relates to a resin foam, which is obtained by uniting at least one member selected from the group consisting of a high water-absorbing resin and particles and fibers using the same.

The present invention also relates to a resin foam in which the water absorption expansion ratio represented by equation (2) when immersed in a physiological sodium chloride solution at 37°C for 24 hours is lower than 50 vol%:

$$\text{Water absorption expansion ratio} = 100 \times (\text{Foam volume after immersion} - \text{Foam volume before immersion}) / (\text{Foam volume before immersion}) \; (2).$$

immersion) (2).

The present invention also relates to a process for producing a resin foam, including mixing a resin composition obtained by adding an alkenyl group-containing compound (A), a hydrosilylation catalyst (C), and a foaming agent (D), and, depending on the case, another additive, and adding and mixing a hydrosilyl group-containing compound (B) for injection foaming or spray foaming.

Effects of the Invention

The present invention provides a resin foam which can be preferably used for medical use and a flexible silicone type resin foam excellent in water absorbing property. More specifically, the present invention is useful for fields requiring a water absorbing property, such as medical use, such as a wound dressing material, a foot care pad, a catheter fixing sheet, a bleeding stop pad, and a care pad, a makeup puff, a toiletry material, an agricultural material, a gardening flooring material, a cleaning material, etc.

Best Modes for Carrying Out the Invention

Resin for use in a resin foam of the present invention contains a siloxane unit and an oxyalkylene unit in the molecular structure and does not contain an isocyanate derived unit. Since a siloxane unit and an oxyalkylene unit are contained in the molecular structure, a foam usable for medical use is obtained in which a balance between moisture permeability or flexibility and compatibility with the skin is excellent; and since an isocyanate derived unit is not contained, a foam usable for medical use is obtained which is free from concerns regarding toxicity of a residual isocyanate. Examples of the oxyalkylene unit include, but not limited thereto, a compound having an alkylene group having 1 to 20 carbon atoms, such as oxymethylene, oxyethylene, oxypropylene, oxyisopropylene, oxybutylene, and oxyisobutylene. It is preferable to contain at least one oxyethylene unit in terms of imparting a water absorbing property to a flexible water absorbing resin foam and imparting properties of absorbing a body fluid or sweat to a foam for medical use. It is more preferable to contain an oxypropylene unit and an oxyethylene unit.

In the water absorbing resin foam of the present invention, the water absorption ratio represented by equation (1) when immersed in a physiological sodium chloride solution at 37°C for 24 hours is preferably 200 wt% or more and lower than 2000 wt%, more preferably 250 wt% or more and lower than 1700 wt%, and still more preferably 300 wt% or more and lower than 1500 wt%:

$$\text{Water absorption ratio} = 100 \times (\text{Foam weight after immersion} - \text{Foam weight before immersion}) / (\text{Foam weight before immersion}) \; (1).$$

When the water absorption ratio is lower than 200 wt%, there is a tendency that sufficient absorption effects cannot be obtained. When the water absorption ratio is 2000 wt% or more, there is a tendency that mechanical properties of a foam decrease at the time of water absorption, making it difficult to handle.

In order to develop the water absorption property, a foam resin contains an oxyethylene unit in a proportion of preferably 5 wt% or more and lower than 80 wt%, more preferably 7 wt% or more and lower than 70 wt%, and still more preferably 10 wt% or more and lower than 60 wt%.

The content of an oxyethylene unit of a foam resin can be calculated from the amount of an oxyethylene unit in a raw material added in preparing resin, and can be measured by a process employing peak intensity derived from a $CH_2O$ group in IR spectrum described in "Kaimenkasseizai Bunsekiho" (Surfactant analytical process), New edition (published by Saiwai Shobo, edited by Kaimenkasseizai Bunseki Kenkyuukai, 1987, p. 282).

There is no limitation on the resin composition of the present invention insofar as the above-mentioned molecular structure is provided. For example, resin obtained by curing a mixture containing an alkenyl group-containing compound (A), a hydrosilyl group-containing compound (B), and a hydrosilylation catalyst (C) is preferable because it is excellent

in expansion moldability, mechanical physical properties, and a balance between the above-mentioned various physical properties as the foam for medical use.

The alkenyl group-containing compound (A) is not limited insofar as it is a compound containing an alkenyl group. It is preferable that the alkenyl group-containing compound (A) be an organic compound containing no siloxane unit in the molecular structure because skin compatibility and the like are excellent and various properties, such as a water absorbing property, can be added.

When the molecular structure of the alkenyl group-containing compound (A) is divided into the skeletal part and an alkenyl group bonded to the skeleton via a covalent bond, the alkenyl group may exist anywhere in the molecule, and preferably exists at the side chain or the terminal in terms of reactivity.

It is preferable that the skeleton of the alkenyl group-containing compound (A) be a usual organic polymer skeleton or an organic monomer skeleton containing no silicon as a structural element and containing carbon alone or carbon and at least one member selected from the group consisting of oxygen, hydrogen, nitrogen, sulfur, and halogen for the above-described reasons. Examples of an organic polymer skeleton include a polyoxyalkylene skeleton, a polyester skeleton, a polycarbonate skeleton, a saturated hydrocarbon skeleton, a polyacrylic acid ester skeleton, a polyamide skeleton, and a phenolformaldehyde (phenol resin) skeleton. Examples of a monomer skeleton include a phenol skeleton, a bisphenol skeleton, or a mixture thereof.

Among the above, a polyoxyalkylene polymer skeleton containing a repeating unit represented by General Formula ($-R^1-O-$) is preferable for obtaining a flexible foam useful as a foam for medical use and having a water absorbing property. Here, $-R^1-$ is a divalent alkylene group. The polyoxyalkylene polymer may contain one kind of repeating unit and a plurality of repeating units. The polyoxyalkylene polymer may be a straight chain polymer or a branched polymer.

Specifically, polyoxyethylene, polyoxypropylene, polyoxy tetramethylene, a polyoxyethylene-polyoxypropylene copolymer, etc., are mentioned. As a foam for medical use, a particularly preferable skeleton is polyoxypropylene, i.e., $-R^1-$ being $-CH_2CH(CH_3)-$, because irritation on the skin is low and wettability to the skin improves to a suitable degree. Moreover, polyoxypropylene is preferable also in terms of availability and workability. In a flexible water absorbing resin foam, when a polyoxyethylene skeleton is not contained in the skeleton of the alkenyl group-containing compound (A), it is preferable to separately introduce polyethylene glycol and its derivatives mentioned later from the viewpoint of giving a water absorbing property.

The alkenyl group-containing compound (A) of the present invention contains a polyoxyalkylene polymer having at least one alkenyl group particularly at the terminal in a proportion of preferably 50 wt% or more, more preferably 70 wt% or more, and still more preferably 80 wt% or more.

The number average molecular weight of a polyoxyalkylene polymer is preferably from 3000 to 50000, more preferably 4000 to 40000, and still more preferably from 5000 to 30000 because workability at room temperature is excellent and excellent skin compatibility is obtained. When the number average molecular weight is lower than 3000, there is a tendency that a foam to be obtained becomes weak and a foam is difficult to manufacture. In contrast, when the number average molecular weight exceeds 50000, there is a tendency that the viscosity increases, lowering workability. The number average molecular weight of a polyoxyalkylene polymer is a number average molecular weight in terms of polystyrene measured by GPC.

There is no limitation on the alkenyl group in the alkenyl group-containing compound (A) of the present invention, insofar as the group contains a carbon-carbon double bond which is active on a hydrosilylation reaction. Examples of the alkenyl group include an unsaturated aliphatic hydrocarbon group having preferably 2 to 20 carbon atoms and more preferably 2 to 6 carbon atoms (e.g., a vinyl group, an allyl group, a methylvinyl group, a propenyl group, a butenyl group, a pentenyl group, and a hexenyl group); an unsaturated cyclic hydrocarbon group having preferably 3 to 20 carbon atoms and more preferably 3 to 6 carbon atoms (e.g., a cyclopropenyl group, a cyclobutenyl group, a cyclopentenyl group, and a cyclohexenyl group); and a methacrylic group.

From the viewpoint of ease of synthesis of introducing an alkenyl group into the skeletal part, the following (1) and (2) are mentioned as a preferable alkenyl group. In the following formulae, $R^2$ is a hydrogen atom or a hydrocarbon group having 1 to 10 carbon atoms, with preferably a hydrogen atom or a methyl group.

(1) $H_2C = C(R^2)-$

(2) $HC(R^2) = CH-$

The average number of alkenyl groups per mol in the alkenyl group-containing compound (A) of the present invention is preferably at least one, more preferably 1 to 5, still more preferably 1 to 3, and particularly preferably 1 to 2. When the average number of alkenyl groups per mol in the alkenyl group-containing compound (A) is lower than 1, there is a tendency that a curing property becomes insufficient. Moreover, depending on the molecular weight of the skeletal part, when the number of alkenyl groups contained in a single molecule is excessively large, the network structure becomes dense, resulting in a tendency that a foam is hardened and becomes weak and that flexibility, mechanical

strength, skin following properties, and texture deteriorate.

The number of the average alkenyl groups per mol is a value measured based on an iodine value. The bonding manner of an alkenyl group to the skeleton is not limited, and a direct linkage, an ether linkage, an ester linkage, a carbonate linkage, a urea linkage, etc., of an alkenyl group are mentioned.

The process for producing the alkenyl group-containing compound (A) of the present invention is not limited. For example, when a polyoxyalkylene polymer is a skeleton, a process involving obtaining a polyoxyalkylene polymer, and then introducing an alkenyl group is mentioned. In this case, for the polyoxyalkylene polymer, various known producing processes can be applied, and a commercially available polyoxyalkylene polymer may be used. Moreover, a process of introducing an alkenyl group into a polyoxyalkylene polymer is also known. For example, a process is mentioned which involves copolymerizing a monomer containing an alkenyl group (e.g., allyl glycidyl ether) and a monomer for synthesizing a polyoxyalkylene polymer or a process is mentioned which involves reacting, with a polyoxyalkylene polymer in which a functional group (e.g., a hydroxy group or an alkoxide group) is introduced beforehand into a desired part (terminal or the like of a main chain), a compound containing both a functional group having reactivity to the functional group and an alkenyl group (e.g., acrylic acid, methacrylic acid, vinyl acetate, or acrylic acid chloride).

There is no limitation on the hydrosilyl group-containing compound (B) of the present invention insofar as it is a compound having a hydrosilyl group. For obtaining a foam, a compound having 1 to 100 hydrosilyl groups in a single molecule is preferable. Here, a hydrosilyl group refers to a group having an Si-H bond.

In the present invention, when two hydrogen atoms (H) are bonded to the same silicon atom (Si), the number of hydrosilyl groups is calculated to be 2.

In the hydrosilyl group-containing compound (B) of the present invention, there is no limitation on the chemical structure other than a hydrosilyl group.

The number average molecular weight of the hydrosilyl group-containing compound (B) of the present invention is preferably 400 to 30000 and more preferably 500 to 10000. When the number average molecular weight of the hydrosilyl group-containing compound (B) is lower than 400, there is a tendency that breaking of bubbles at the time of foaming is noticeable, making it difficult to obtain a foam. When the number average molecular weight exceeds 30000, there is a tendency that a curing rate is low and manufacturing efficiency becomes low.

The number of hydrosilyl groups contained in a single molecule of the hydrosilyl group-containing compound (B) is preferably 1 to 100, and it is preferable to contain a larger number of hydrosilyl groups insofar as compatibility with other components is impaired. When the number of hydrosilyl groups contained in a single molecule of the compound (B) is 2 or more, a plurality of compound (A) molecules can be cross-linked in curing. When the number of hydrosilyl groups contained in a single molecule of the compound (B) is lower than 2, a curing rate is low, resulting in poor curing in many cases. Moreover, as described later, when an active hydrogen-containing compound is used as a foaming agent (D), the compound (B) and the active hydrogen compound undergo dehydrogenation condensation to be involved in foaming. Thus, depending on a target expansion ratio, the number of the hydrosilyl groups generally preferably exceeds 2.

Moreover, for the same reasons, the hydrosilyl group-containing compound (B) in the present invention has preferably 2 mol equivalent or more of a hydrosilyl group, relative to the alkenyl group in the alkenyl group-containing compound (A).

When the number of hydrosilyl groups in the hydrosilyl group-containing compound (B) is excessively large, cross-linking becomes excessively dense, resulting in that flexibility and skin following properties of the obtained foam are likely to decrease, and further, stability of the compound (B) deteriorates. Furthermore, when an excessively amount of hydrosilyl groups remains in the obtained foam, the remaining hydrosilyl groups cause skin irritation or a void. Moreover, roughness and denseness of cross-linking also has an influence on moisture permeability or a water absorbing property.

Therefore, the number of the hydrosilyl groups in the hydrosilyl group-containing compound (B) is selected in consideration of a balance between the number of the alkenyl groups of the compound (A) and the number of functional groups in another additive which reacts with the hydrosilyl group, such as an active hydrogen compound. Then, the hydrosilyl group-containing compound (B) in the present invention contains a hydrosilyl group in the amount of preferably 0.1 mol equivalent or more and 50 mol equivalent or lower, more preferably 0.2 mol equivalent or more and 30 mol equivalent or lower, and particularly preferably 0.5 mol equivalent or more and 20 mol equivalent or lower, based on the sum of the alkenyl group(s) of the compound (A) and the functional group(s) present in another additive and capable of reacting with the hydrosilyl group.

The hydrosilyl group-containing compound (B) in the present invention may be used singly or in combination of two or more.

The hydrosilyl group-containing compound (B) of the present invention preferably has favorable compatibility with the alkenyl group-containing compound (A). As the compound (B) having a suitable hydrosilyl group, organohydrogen siloxane is preferably mentioned in terms of ease of obtaining a raw material and compatibility with the alkenyl group-containing compound (A).

A typical example of organohydrogen siloxane includes a compound represented by General Formula (3) or (4).

[Chemical Formula 1]

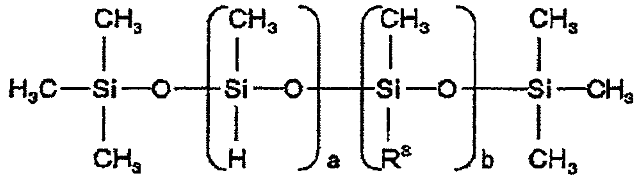

(3)

[Chemical Formula 2]

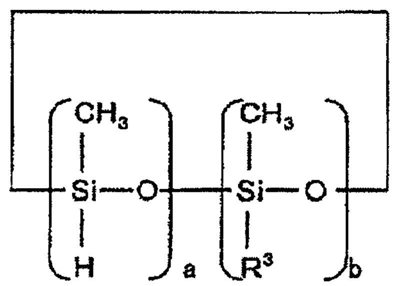

(4)

The value of a of General Formula (3) or (4) is in agreement with the number of the hydrosilyl groups in the molecule. In the formulae, the value of a is 1 or more; the value of b is 0 or more; and the value of a + b is not limited, and is preferably 1 to 100. $R^3$ is not limited, and is preferably at least one member selected from a hydrocarbon group having 2 to 20 carbon atoms in the main chain and a polyoxyalkylene group.

The compound represented by General Formula (3) or (4) can be obtained by introducing unmodified methyl hydrogen silicone itself or modifying methyl hydrogen silicon by introducing $R^3$. Here, the unmodified methyl hydrogen silicone is equivalent to the compound in which all $R^3$s are H in General Formula (3) and is used as a raw material of various kinds of modified silicone as described in "Silicone no shijotenbo-meka senryaku to oyo tenkai-" published by CMC (1990. 1.31)". Examples of an organic compound for introduction of $R^3$ include $\alpha$-olefin, styrene, $\alpha$-methyl styrene, allyl alkyl ether, allyl alkyl ester, allyl phenyl ether, allyl phenyl ester, and polyoxyalkylene allyl ether. With the amount of the above-mentioned organic compound which is added for modification, the number of the hydrosilyl groups in the molecule after modification can be adjusted.

There is no limitation on the quantitative ratio between the compound (A) and the compound (B) for forming the foam of the present invention. As described above, the ratio is expressed by the total amount of the hydrosilyl groups derived from the compound (B) based on the total amount of the alkenyl groups derived from the compound (A). As described above, the compound (B) in the present invention has preferably 2 mol equivalent or more of the hydrosilyl group relative to the alkenyl group of the compound (A). In more detail, the amount is determined according to the type of a foaming agent to be used and a foaming process. The quantitative ratio between the compound (A) and the compound (B) is determined according to the amount of the above-mentioned functional group. When expansion moldability is taken into consideration, it is preferable that the (A)/(B) weight ratio between the compound (A) and the compound (B) is preferably 0.05 or more and 20 or lower and more preferably 0.1 or more and 10 or lower.

There is no limitation on the hydrosilylation catalyst (C) in the present invention, and any hydrosilylation catalyst can be used insofar as it promotes a hydrosilylation reaction. Specific examples of the hydrosilylation catalyst (C) include chloroplatinic acid, a platinum-vinyl siloxane complex (e.g., a platinum-1,3-divinyl-1,1,3,3-tetramethyl disiloxane complex and a platinum-1,3,5,7-tetravinyl 1,3,5,7-tetramethyl cyclotetrasiloxane complex) and a platinum-olefin complex (e.g., $Pt_p(ViMe_2SiOSiMe_2Vi)_qPt[(MeViSiO)_4]r$ (wherein, p, q, and r represent a positive integer and Vi represents a vinyl group)). Among the above, in terms of catalytic activity, a platinum complex catalyst containing no conjugate base of strong acid as a ligand is preferable, a platinum-vinyl siloxane complex is more preferable, and a platinum-1,3-divinyl-1,1,3,3-tetramethyl disiloxane complex or a platinum 1,3,5,7-tetravinyl-1,3,5,7-tetramethyl cyclotetrasiloxane complex is particularly preferable.

The amount of the hydrosilylation catalyst (C) used in the present invention is not particularly limited, but preferably $10^{-8}$ to $10^{-1}$ mol and more preferably $10^{-6}$ to $10^{-3}$ mol based on the total amount of 1 mol of the alkenyl group (s) of the compound (A). When the use amount of the hydrosilylation catalyst (C) is within the above-mentioned range, securing of a suitable curing rate, a stable curing property, a required pot life, etc., are easily achieved.

There is no limitation on a process for producing a flexible water absorbing resin foam in the present invention. A process involving obtaining resin containing a siloxane unit and an oxyalkylene unit in the molecular structure and containing no isocyanate derived unit, and adding a foaming agent (D) to the resin, and heating the mixture for foaming or a process involving adding a foaming agent (D) under pressure, and then releasing the pressure for foaming can be applied. A process involving blending a foaming agent (D) in a mixture of alkenyl group-containing compound (A), hydrosilyl group-containing compound (B), and hydrosilylation catalyst (C), and foaming the mixture simultaneously with curing is preferable in terms of expansion moldability or production efficiency

Examples of the foaming agent (D) in the present invention include, but not limited thereto, a volatile physical foaming agent, a chemical foaming agent which generates gas by thermal decomposition or a chemical reaction, an active hydrogen group-containing compound which reacts with a hydroxyl group to generate hydrogen, etc., for use in an organic foam, such as polyurethane, phenol, polystyrene, and polyolefine. Among the above, the active hydrogen group-containing compound is preferably used because the active hydrogen group-containing compound contributes to improve an open cell coefficient or develop properties as a foam for medical use, such as flexibility.

There is no limitation on the active hydrogen group-containing compound insofar as it is a compound containing an active hydrogen group which reacts with hydroxyl group to generate hydrogen. From the viewpoint of imparting flexibility and a water absorbing property, preferable is not an OH group-containing polysiloxane for use in a silicone foam but a compound in which oxygen is directly bonded to carbon, or water.

As the active hydrogen-containing compound in which oxygen is directly bonded to carbon, a saturated hydrocarbon alcohol, carboxylic acid, or water is preferably used. Specific examples include: water; monovalent alcohols, such as methanol, ethanol, n-propanol, iso-propanol, n-butanol, iso-butanol, tert-butanol, ethylene glycol monomethyl ether, ethylene glycol monoethyl ether, ethylene glycol monopropyl ether, ethylene glycol monobutyl ether, diethylene glycol monomethyl ether, ethylene glycol monophenyl ether, ethylene glycol monoallyl ether, and glycerol diallyl ether; polyhydric alcohols, such as ethylene glycol, propylene glycol, 1,4-butylene glycol, 1,3-butylene glycol, 2,3-butylene glycol, diethylene glycol, triethylene glycol, neopentyl glycol, 1,6-hexamethylene glycol, 1,9-nonamethyleneglycol, glycerol, trimethylolpropane, pentaerythritol, sorbitol, sucrose, and glycerol monoallyl ether; polyether polyols (including substances containing 3 or more of OH groups in the molecule and containing sorbitol, sucrose, tetraethylenediamine, ethylenediamine, etc., as an initiator), such as polypropylene glycol, polyethylene glycol, copolymers thereof, and polytetramethylene glycol; polyester polyols, such as adipate polyol, polycaprolactone polyol, and polycarbonate polyol; epoxy-modified polyols; polyether ester polyol; phenolic polyols, such as benzilic ether phenolic polyol; fluorine polyols, such as Lumiflon (manufactured by Asahi Glass Co., Ltd.); polybutadiene polyols; hydrogenated polybutadiene polyols; castor oil polyols; halogen-containing flame retardant polyols; phosphoric acid-containing flame retardant polyols; carboxylic acids including monovalent saturated carboxylic acids, such as acetic acid and propionic acid; compounds having a phenolic OH group, such as phenol, cresol, xylenol, resorcinol, catechol, pyrogallol, bisphenol A, bisphenol B, bisphenol S, and phenolic resin; OH group-containing vinyl monomers [which can also be used as a combination substance of the compound (A) and the foaming agent (D)], such as 2-hydroxyethyl(meth)acrylate, 2-hydroxypropyl(meth)acrylate, 2-hydroxyethyl vinyl ether, N-methylol(meth)acrylamide, Aronix 5700 manufactured by Toagosei Chemical Industry Co., Ltd., 4-hydroxystyrene, HE-10, HE-20, HP-10, and HP-20 manufactured by Nippon Shokubai Kagaku Kogyo Co., Ltd. [each of which is an acrylic acid ester oligomer containing an OH group at the terminal], Blenmer PP series manufactured by Nippon Oil & Fats Co., Ltd. [polypropylene glycol methacrylate], Blenmer PE series [polyethylene glycol monomethacrylate], Blenmer PEP series [polyethylene glycol polypropylene glycol methacrylate], Blenmer AP-400 [polypropylene glycol monoacrylate], Blenmer AE-350 [polyethylene glycol monoacrylate], Blenmer NKH-5050 [polypropylene glycol polytrimethylene monoacrylate], and Blenmer GLM [glycerol monomethacrylate], and ε-caprolactone-modified hydroxyalkyl vinyl monomer obtained by a reaction between an OH group-containing vinyl compound and ε-caprolactone; acrylic resin having an OH group which can be obtained by copolymerization of the OH group-containing vinyl monomer with acrylic acid, methacrylic acid, derivatives thereof, etc.; and resin having an OH group, such as alkyd resin and epoxy resin.

Among the active hydrogen group-containing compounds, at least one member selected from the group consisting of water, primary alcohol, and polyether polyol is preferable from the viewpoint of reactivity or handling property, and water, ethanol, and polyethylene glycol are more preferable from the viewpoint that it has little effect on the human body when the obtained foam is used for medical use. When polyethylene glycol is used, a water absorbing property can be given to a foam, and thus polyethylene glycol is particularly preferably used.

The hydroxyl equivalent in the active hydrogen group-containing compound in the present invention is preferably 0.1 mmol/g or more because, when the hydroxyl equivalent decreases, the volume of an active hydrogen group-containing compound to be added increases, and thus the expansion ratio does not increase, and more preferably 0.5 mmol/g or more further in terms of reactivity.

In the present invention, in order to easily perform dehydrogenation with the hydrosilyl group in the hydrosilyl group-containing compound (B), carboxylic acids, such as acetic acid and propionic acid, can also be used. For adjustment of an expansion rate, two or more kinds of active hydrogen group-containing compounds can be used in combination.

Further for adjustment of physical properties, such as a cross linking degree, expansion moldability, a water absorbing property, etc., compounds having both a carbon-carbon double bond which can be hydrosilylated and an OH group in the molecule, such as ethylene glycol monoallyl ether, polyethylene glycol monoallyl ether, polypropylene glycol monoallyl ether, monoallyl ether of an ethylene glycol propylene glycol copolymer, glycerol monoallyl ether, glycerol diallyl ether, pentaerythritol diallyl ether, and pentaerythritol triallyl ether can also be used.

When an active hydrogen compound having two or more OH groups in a single molecule or an active hydrogen compound having both an OH group and an alkenyl group in a single molecule is used, hydrogen gas generates due to a reaction between the hydrosilyl group in the hydrosilyl group-containing compound (B) and the OH group in an active hydrogen compound and a crosslinking structure formed by a reaction between the hydrosilyl group with the OH group and the alkenyl group is formed. Thus, using a large amount of the active hydrogen compound is not preferable because curing may occur before sufficient foaming.

When an active hydrogen compound is used as the foaming agent (D) in the present invention, the proportion of each of the compound (A), the compound (B), and the foaming agent (D) is suitably selected without limitation according to the structure of each compound, a target expansion ratio, and target physical properties. The ratio between the number of moles x of the hydrosilyl group in the compound (B) and the sum of the number of moles y of the alkenyl group in the compound (A) and the number of moles z of the OH group in the foaming agent (D) is preferably x : y + z = 50:1 to 1:10, more preferably x: y + z = 30:1 to 1:5, and still more preferably x: y + z = 20:1 to 1:2. When the molar ratio of the hydroxyl group exceeds x : y + z = 50:1, the crosslinking density becomes low, resulting in a tendency that sufficient mechanical strength is not obtained. When the molar ratio is lower than x: y+z =1:10, sufficient foaming and curing may not occur.

The ratio between the number of moles y of the alkenyl group in the compound (A) and the number of moles z of the OH group in the foaming agent (D) is not limited, and can be suitably selected according to a target expansion ratio, target physical properties, the skeleton of the compound (A), and the type of the foaming agent (D). In general, y: z = 100:1 to 1:100 is preferable and y:z = 10:1 to 1:20 is more preferable.

As the foaming agent (D), the above-mentioned physical foaming agents and chemical foaming agents may be used singly or in combination with an active hydrogen compound besides the above-mentioned active hydrogen compounds.

The physical foaming agent is not limited insofar as a hydrosilylation reaction is not impeded. From the viewpoint of foaming property, workability, and safety, a compound having a boiling point of 100˚C or lower is preferable, and a compound having a boiling point of 50˚C or lower is more preferable. Specifically, organic compounds, such as hydrocarbon, chlorofluorocarbon, alkyl chloride, and ether, and inorganic compounds, such as carbon dioxide, nitrogen, and air, are mentioned. From the viewpoint of environmental compatibility, it is preferable to use a compound selected from hydrocarbon, ether, carbon dioxide, nitrogen, and air. Among the above, examples of hydrocarbon include methane, ethane, propane, n-butane, isobutane, n-pentane, isopentane, neopentane, n-hexane, 2-methylpentane, 3-methylpentane, 2,2-dimethylbutane, 2,3-dimethylbutane cyclobutane, cyclopentane, and cyclohexane. Examples of ethers include dimethyl ether, diethyl ether, ethylmethyl ether, dipropyl ether, diisopropyl ether, butylmethyl ether, butylethyl ether, tert-butylmethyl ether, tert-butylethyl ether, and 1,1-dimethyl propylmethyl ether. When mechanical stirring is performed in the air in manufacturing a foam, bubbles may be formed due to air entrained during stirring, the air which is considered to be one of the physical foaming agents. In the case where the physical foaming agents are used for medical use, for example, effects of a residual substance of the physical foaming agent on the human body need to be considered in some cases. After manufacturing a foam, it is preferable that the obtained foam be cured by heating at a temperature higher than the boiling point of the used physical foaming agent to remove the residual substance of the physical foaming agent.

The chemical foaming agent other than the active hydrogen compound is not limited insofar as a hydrosilylation reaction is not impaired. For example, inorganic chemical foaming agents, such as $NaHCO_3$, $(NH_4)_2CO_3$, $NH_4HCO_3$, $NH_2NO_2$, $Ca(N_3)_2$, and $NaBH_4$ and organic chemical foaming agents, such as azodicarbonamide, azobisisobutyronitril, barium azodicarboxylate, dinitrosopentamethylenetetramine, and paratoluenesulfonylhydrazide are mentioned. In the case where the chemical foaming agents are used for medical use, for example, effects of a residual substance of the chemical foaming agent on the human body need to be considered in some cases. Thus, the use thereof is limited.

To the foam of the present invention, a filler, an anti-aging agent, a radical inhibitor, a UV absorber, an adhesion improving agent, a flame retardant, a foam adjusting agent, such as polydimethylsiloxane polyalkylene oxide surfactants or organic surfactants (e.g., polyethylene glycol alkylphenyl ether), an acid compound or a basic compound (which is an additive for adjusting a reaction between a hydrosilyl group and an OH group, in which condensation reaction is suppressed by an acid and accelerated by a base), a storage stability improving agent, an antiozonant, a light stabilizer, a thickener, a plasticizer, a coupling agent, an antioxidant, a thermostabilizer, an electrical conductivity imparting agent,

an antistatic agent, a radiation screening agent, a nucleating agent, a phosphorus peroxide decomposer, a lubricant, a pigment, a metal deactivator, a physical-property controlling agent, etc., can be added insofar as the objects and effects of the present invention are not impaired. Since the present invention is applied for medical use, the use thereof may be limited.

In the present invention, among the above, at least one member selected from the group consisting of a high water absorbing resin and particles and fibers using the same can be added for the purpose of increasing a water absorbing property or a water absorbing rate described later.

Since the present invention is applied for medical use, the use thereof may be limited. Specific examples of a high water-absorbing resin include natural polysaccharides, carboxymethylcellulose (CMC), alginic acid, alginate, polyacrylic acid, polyacrylamide, polyacrylate, polymethacrylate, polyacrylonitrile, polyvinyl pyrrolidone, polyvinyllactam, polyvinylpyridine, polyvinyl alcohol, polyvinyl acetate, polyethylene oxide, gelatin, or another hydrophilic polypeptide, carrageenan, pectin, xanthene, chitin, chitosan, starch, and salts thereof, derivatives, copolymers, such as a starch-acrylic acid graft copolymer, a vinyl alcohol-acrylate copolymer, an ethylene-vinyl alcohol copolymer, and a polyacrylonitrile-methyl methacrylate-butadiene copolymer, and mixtures thereof.

There is no limitation on a process of uniting a high water-absorbing resin and particles and fibers using the same with a foam. A process involving laminating a foam on a high water-absorbing resin and particles and fibers using the same or a process involving blending a high water-absorbing resin and particles and fibers using the same in a foamable resin composition, and then obtaining a foam is mentioned.

As an additive contributing to the improvement in a water absorbing property, fine particles having a particle diameter of 1000 nm or lower and having a hydroxyl group on the surface, such as anhydrous silica (silicon oxide) having a silanol group on the surface, layer silicate with a water absorbing property, such as smectite, and expandable fluorine mica or organification-treated articles thereof, a porous material, such as zeolite, activated carbon, alumina, silica gel, porous glass, activated clay, and diatomite, etc., may be added.

Moreover, a surfactant can also be added for the purpose of improving foam stabilizing property and compatibility of compounds (A) to (D). The type of the surfactant is not limited, and specific examples include alkyl sulfate, such as sodium lauryl sulfate, polyoxyethylene alkyl ether sulfate, such as polyoxyethylene lauryl ether sodium sulfate, polyoxyethylene alkyl ether acetate, lauryl trimethyl ammonium chloride, alkoxy propyl trimethylammonium chloride, dialkyl dimethyl ammonium chloride, a benzalkonium chloride solution, alkyl dimethylamino acetic acid betaine, alkyl dimethyl amine oxide, alkyl carboxymethyl hydroxyethyl imidazolium betaine, alkylamide propyl betain, glycerol fatty acid ester, propylene glycol fatty acid ester, sorbitan fatty acid ester, and like nonionic surfactants.

The density of the foam of the present invention is not limited, and is preferably 10 kg/$m^3$ or more and lower than 500 kg/$m^3$, and more preferably 20 kg/$m^3$ or more and lower than 400 kg/$m^3$. When the density is lower than 10 kg/$m^3$, there is a tendency that a mechanical property decreases, and a handling property is bad. When the density is 500 kg/$m^3$ or more, there is a tendency that foaming property, such as flexibility, are not obtained.

The open cell coefficient of the foam of the present invention is not limited, and is preferably 80% or more, and more preferably 90% or more. When the open cell coefficient is lower than 80%, there is a tendency that properties suitable as a foam for medical use, such as flexibility or texture, are difficult to obtain the water absorbing property mentioned later. The open cell coefficient is measured according to ASTM D2856 (1998).

The thickness of the foam of the present invention is preferably 1 mm or more and lower than 100 mm. When the thickness is lower than 1 mm, sufficient functions as a foam cannot be exhibited. When the thickness is 100 mm or more, handling when used for medical use becomes difficult.

Also in a foam for medical use of the present invention, the water absorption represented by equation (1) when immersed in a physiological sodium chloride solution at 37°C for 24 hours is preferably 200 wt% or more and lower than 2000 wt%, more preferably 250 wt% or more and lower than 1700 wt%, and still more preferably 300 wt% or more and lower than 1500 wt%:

$$\text{Water absorption} = 100 \times (\text{Foam weight after immersion - Foam weight before immersion}) / (\text{Foam weight before immersion}) \quad (1).$$

It is preferable to adjust the water absorption expansion ratio represented by equation (2) when immersed in a physiological sodium chloride solution at 37°C for 24 hours to be lower than 50 vol%, because the foam of the present invention is excellent in a favorable sweat absorbing property and a body fluid absorbing property and can be preferably used for a wound dressing material and the like:

$$\text{Water absorption expansion ratio} = 100 \times (\text{Foam volume after immersion} - \text{Foam volume before immersion}) / (\text{Foam volume before immersion}) \quad (2).$$

When the water absorption is lower than 200 wt%, sufficient sweat and body fluid absorbing effects are not obtained. When the water absorption is 2000 wt% or more, mechanical property of a foam decrease upon water absorption to make it difficult to handle the foam, resulting in that the foam is not practically used.
The water absorption is more preferably 250 wt% or more and lower than 1700 wt% and particularly preferably 300 wt% or more and 1500 wt% or lower.
When the water absorption expansion ratio is 50 vol% or more, oppressive feeling or uncomfortable feeling develops due to expansion. Thus, such a water absorption expansion ratio is not preferable. The water absorption expansion ratio is more preferably lower than 40 vol%, and particularly preferably lower than 30 vol%.
In order to develop the above-mentioned water absorbing property, a foam resin contains an oxyethylene unit in a proportion of 5 wt% or more and lower than 80 wt%, more preferably 7 wt% or more and lower than 70 wt%, and still more preferably 10 wt% or more and lower than 60 wt%.
There is no limitation on the shape of the foam of the present invention. For example, foams formed into a plate shape, a sheet shape, a mass of indefinite shape, a bead shape, a bag-like shape, or a shape of clothes are mentioned, and foams formed into a sheet are widely used. The foam of the present invention may be used singly or may be integrally molded with a material, such as film, cloth, a nonwoven fabric, or paper for use. Moreover, when the foam of the present invention is directly adhered to the skin for use similarly as in the case of the above-mentioned wound dressing material, the foam of the present invention can also be united with a binder, a self-adhesive film, a bandage, etc., for use.
The foam of the present invention may be used while leaving the surface skin layer formed at the time of expansion molding. Or, the surface skin layer may be cut off for use or may be cut into a desired form for use. However, when the foam is used for applications requiring to effectively develop the above-described water absorbing property, such as a wound dressing material, the surface skin layer needs to be cut off or an opening part needs to form on the surface skin layer.
There is no limitation on a process for producing the foam of the present invention. A process is preferably used which involves mixing a resin composition obtained by adding the alkenyl group-containing compound (A), the hydrosilylation catalyst (C), the foaming agent (D), and, depending on the case, another additive, adding and mixing the hydrosilyl group-containing compound (B), and performing injection foaming or spray foaming to mold the mixture into a desired form as described above.

Examples

Hereinafter, the foam of the present invention will be described in more detail with reference to examples, but the present invention is not limited only to the examples. In the following examples and comparative examples, "part" represents "part by weight" and "%" represents "% by weight" unless otherwise specified. In the examples, the following compounds were used.

A: Alkenyl group-containing compound

A-1: Allyl-terminated polyoxyalkylene (see the following synthesis examples, alkenyl group content: 0.219 mmol/g)

B: Hydrosilyl group-containing compound

B-1: KF-99 (methyl hydrogen silicone oil, manufactured by Shin-Etsu Chemical Co., Ltd., hydrosilyl group content: 16.6 mmol/g)

C: Hydrosilylation catalyst

C-1: Platinum-1,3-divinyl-1,1,3,3-tetramethyldisiloxane complex (3% by weight platinum isopropanol solution)

D: Foaming agent

D-1: Ethanol (OH group content: 21.7 mmol/g)
D-2: Polyethylene glycol (Macrogol 400, manufactured by Sanyo Chemical Industries, Ltd., molecular weight: 400, OH group content: 5.00 mmol/g)
D-3: Polyethylene glycol monoallyl ether (Uniox PKA-5002, manufactured by Nippon Oil & Fats Co., Ltd., alkenyl group content: 2.50 mmol/g, OH group content: 2.50 mmol/g)

D-4 : Isopentane
E: Other raw materials

E-1: Moisture permeable polyurethane film (DSU-214-CDB, manufactured by Sheedom Co., Ltd., 30 μ (basis weight: 35 $g/m^2$)) A synthesis example of compound A-1 will be described below.

(Synthesis of compound A-1)

Oxypropylene polymer glycol having a number average molecular weight of 3000 was obtained by a polymerization process using caustic alkali. According to the process of synthesis-example 1 of Japanese Unexamined Patent Publication No. 5-117521, propylene oxide was polymerized using a composite metal cyanide complex catalyst (zinc hexacyanocobaltate) and using the oxypropylene polymer glycol as an initiator, thereby obtaining a polymer having a number average molecular weight of 13800. A 28% methanol solution of sodium methylate and allyl chloride were used to the polymer to convert the terminal to an allyl group, and the resultant was subjected to purification by desalting, thereby obtaining a polyoxyalkylene compound (compound A-1) having generally two allyl terminals in a single molecule. The amount of the allyl-terminated group of the obtained polymer was 0.219 mmol/g.

(Example 1)

To 100 parts by weight of compound (A-1), 7 parts by weight of ethanol (D-1) as a foaming agent, 22 parts by weight of polyethylene glycol (D-2), 0.3 part by weight of hydrosilylation catalyst (C-1) were added, the mixture was sufficiently mixed, 13 parts by weight of compound (B-1) was further added, and the mixture was quickly mixed. The resultant was allowed to stand at room temperature over night, thereby obtaining a foam. The density of the obtained foam was 120 $kg/m^3$ and the open cell coefficient thereof was 100%. The measurement results of the obtained foam are shown in Table 1.

(Example 2)

To 100 parts by weight of compound (A-1), 7 parts by weight of ethanol (D-1) as a foaming agent, 22 parts by weight of polyethylene glycol monoallyl ether (D-3), and 0.6 part by weight of hydrosilylation catalyst (C-1) were added, the mixture was sufficiently mixed, 13 parts by weight of compound (B-1) was further added, and the mixture was quickly mixed. The resultant was allowed to stand at room temperature over night, thereby obtaining a foam. The density of the obtained foam was 60 $kg/m^3$ and the open cell coefficient thereof was 100%. The measurement results of the obtained foam are shown in Table 1.

(Example 3)

To 100 parts by weight of compound (A-1), 7 parts by weight of ethanol (D-1) as a foaming agent, 22 parts by weight of polyethylene glycol (D-2), 5 parts by weight of isopentane (D-4), and 0.3 part by weight of hydrosilylation catalyst (C-1) were added, the mixture was sufficiently mixed, 13 parts by weight of compound (B-1) was further added, and the mixture was quickly mixed. The resultant was allowed to stand at room temperature over night, thereby obtaining a foam. The density of the obtained foam was 105 $kg/m^3$ and the open cell coefficient thereof was 100%. The measurement results of the obtained foam are shown in Table 1.

(Comparative Example 1)

To 100 parts by weight of compound (A-1), 12 parts by weight of ethanol (D-1) as a foaming agent and 0.3 part by weight of hydrosilylation catalyst (C-1) were added, the mixture was sufficiently mixed, 13 parts by weight of compound (B-1) was further added, and the mixture was quickly mixed. The resultant was allowed to stand at room temperature over night, thereby obtaining a foam. The density of the obtained foam was 100 $kg/m^3$ and the open cell

coefficient thereof was 96%. The measurement results of the obtained foam are shown in Table 1.

(Comparative Example 2)

15 parts by weight of 2,6-tolylenediisocyanate (molecular weight: 174.16), 85 parts by weight of polyethylene glycol (Macrogol 1500, manufactured by Sanyo Chemical Industries, Ltd., molecular weight: 1500), 0.1 part by weight of triethylamine, and 4 parts by weight of water were sufficiently stirred. The resultant was allowed to stand at room temperature over night, thereby obtaining a foam. The density of the obtained foam was 130 kg/$m^3$ and the open cell coefficient thereof was 100%. The measurement results of the obtained foam are shown in Table 1.

[Table 1]

| | | Example1 | Example2 | Example3 | Comparative Example1 | Comparative Example2 |
|---|---|---|---|---|---|---|
| Presence of isocyanate | | None | None | None | None | Present |
| mol equivalent ratio | Alkenyl group in compound (A) | 1 | 1 | 1 | 1 | |
| | Hydrosilyl group in compound (B) | 10 | 10 | 10 | 10 | |
| | OH group in foaming agent (D) | 12 | 9 | 12 | 12 | |
| | Alkenyl group in foaming agent (D) | | 3 | | | |
| Oxyethylene content in foam resin | | 15 | 14 | 15 | 0 | 82 |
| Density (kg/m3) | | 120 | 60 | 105 | 100 | 130 |
| Open cell coefficient (%) | | 100 | 100 | 100 | 96 | 100 |
| Water absorption (wt%) | | 600% | 1000% | 700% | 30% | 1200% |
| Hardness (N) | | 1.4 | 1.1 | 1.3 | 1.8 | 2.2 |

The above results reveal that the present invention provides a flexible water absorbing resin foam containing a siloxane unit and an oxyalkylene unit in the molecular structure and containing no isocyanate derived unit.

(Example 4)

To 100 parts by weight of compound (A-1), 7 parts by weight of ethanol (D-1) as a foaming and 0.3 part by weight of hydrosilylation catalyst (C-1) were added, and sufficiently mixed. Then, 13 parts by weight of compound (B-1) was further added, and quickly mixed. Then, the resultant was uniformly applied to separate paper which was subjected to separation treatment with a thickness of 3 mm using an applicator. The upper surface was covered with separate paper which was subjected to separation treatment through a 6 mm thick spacer. The resultant was put in a press, allowed to stand at room temperature for 10 minutes, and then heated at 120 ˚C for 5 minutes. The obtained foam sheet was sliced in half in the thickness direction with a slicer, thereby obtaining a 3 mm thick foam sheet with a surface skin at one side. The density was 130 kg/$m^3$ and the open cell coefficient was 100%.

(Example 5)

To 100 parts by weight of compound (A-1), 7 parts by weight of ethanol (D-1) as a foaming, 22 parts by weight of polyethylene glycol (D-2), and 0.3 part by weight of hydrosilylation catalyst (C-1) were added, and sufficiently mixed. Then, 13 parts by weight of compound (B-1) was further added, and quickly mixed. Then, the resultant was uniformly

applied to separate paper which was subjected to separation treatment with a thickness of 3 mm using an applicator. The upper surface was covered with separate paper which was subjected to separation treatment through a 6 mm thick spacer. The resultant was put in a press, allowed to stand at room temperature for 10 minutes, and then heated at 120˚C for 5 minutes. The obtained foam sheet was sliced in half in the thickness direction with a slicer, thereby obtaining a 3 mm thick foam sheet with a surface skin at one side. The density was 129 kg/$m^3$ and the open cell coefficient was 100%.

(Example 6)

To 100 parts by weight of compound (A-1), 7 parts by weight of ethanol (D-1) as a foaming agent, 22 parts by weight of polyethylene glycol (D-2), 0.3 part by weight of hydrosilylation catalyst (C-1) were added, and sufficiently mixed. Then, 13 parts by weight of compound (B-1) was further added, and quickly mixed. Then, the resultant was uniformly applied to a moisture permeable polyurethane film (E-1) (DSU-214-CDB, manufactured by Sheedom Co., Ltd., 30 μ) (basis weight: 35 g/$m^2$) with a thickness of 3 mm using an applicator. The upper surface was covered with a moisture permeable polyurethane film (E-1) through a 6 mm thick spacer. The resultant was put in a press, allowed to stand at room temperature for 10 minutes, and then heated at 120˚C for 5 minutes. The obtained foam sheet was sliced in half in the thickness direction with a slicer, thereby obtaining a 3 mm thick foam sheet with the moisture permeable polyurethane film at one side. The density was 150 kg/$m^3$ and the open cell coefficient was 95%.

(Example 7)

To 100 parts by weight of compound (A-1), 7 parts by weight of ethanol (D-1) as a foaming, 22 parts by weight of polyethylene glycol (D-2), and 0.3 part by weight of hydrosilylation catalyst (C-1) were added, and sufficiently mixed. Then, 13 parts by weight of compound (B-1) was further added, and quickly mixed. Then, the resultant was uniformly applied to separate paper which was subjected to separation treatment with a thickness of 2 mm using an applicator. The upper surface was covered with separate paper which was subjected to separation treatment through a 3 mm thick spacer. The resultant was put in a press, allowed to stand at room temperature for 10 minutes, and then heated at 120˚C for 5 minutes. One side of the obtained 3 mm thick foam sheet with the surface skin at both sides was perforated using CO2 laser-marker LP-200 manufactured by SUNX to form a through hole penetrating the skin layer of one side having a hole diameter of 500 μ in such a manner that the opening ratio thereof was 20%. The density was 200 kg/$m^3$ and the open cell coefficient was 90%.

(Example 8)

To 100 parts by weight of compound (A-1), 7 parts by weight of ethanol (D-1) as a foaming agent, 22 parts by weight of polyethylene glycol monoallyl ether (D-3), and 0.6 part by weight of hydrosilylation catalyst (C-1) were added, and sufficiently mixed. Then, 13 parts by weight of compound (B-1) was further added, and quickly mixed. Then, the resultant was uniformly applied to separate paper which was subjected to separation treatment with a thickness of 3 mm using an applicator. The upper surface was covered with separate paper which was subjected to separation treatment through a 6 mm thick spacer. The resultant was put in a press, allowed to stand at room temperature for 10 minutes, and then heated at 120˚C for 5 minutes. The obtained foam sheet was sliced in half in the thickness direction with a slicer, thereby obtaining a 3 mm thick foam sheet with a surface skin at one side. The density was 65 kg/$m^3$ and the open cell coefficient was 100%.

(Example 9)

To 100 parts by weight of compound (A-1), 7 parts by weight of ethanol (D-1) as a foaming, 22 parts by weight of polyethylene glycol (D-2), 5 parts by weight of isopentane (D-4), and 0.3 part by weight of hydrosilylation catalyst (C-1) were added, and sufficiently mixed. Then, 13 parts by weight of compound (B-1) was further added, and quickly mixed. Then, the resultant was uniformly applied to separate paper which was subjected to separation treatment with a thickness of 3 mm using an applicator. The upper surface was covered with separate paper which was subjected to separation treatment through a 6 mm thick spacer. The resultant was put in a press, allowed to stand at room temperature for 10 minutes, and then heated at 120˚C for 5 minutes. The obtained foam sheet was sliced in half in the thickness direction with a slicer, thereby obtaining a 3 mm thick foam sheet with a surface skin at one side. The density was 110 kg/$m^3$ and the open cell coefficient was 100%.

(Comparative Example 3)

15 parts by weight of 2, 6-TDI (molecular weight: 174.16), 85 parts by weight of Macrogol 1500 (molecular

weight: 1500), 0.1 part by weight of triethylamine, and 4 parts by weight of water were sufficiently stirred. Then, the resultant was uniformly applied to a moisture permeable polyurethane film (DSU-214-CDB, manufactured by Sheedom Co., Ltd., 30 μ (basis weight: 35 g/$m^2$)) with a thickness of 3 mm using an applicator. The upper surface was covered with a moisture permeable polyurethane film (E-1) through a 6 mm thick spacer. The resultant was put in a press, allowed to stand at room temperature for 10 minutes, and then heated at 120˚C for 5 minutes. The obtained foam sheet was sliced in half in the thickness direction with a slicer, thereby obtaining a 3 mm thick foam sheet with the moisture permeable polyurethane film at one side. The density was 140 kg/$m^3$ and the open cell coefficient was 100%.

The above measurement results in each foam sheet were shown together in Table 1.

[Table 2]

| | | Example4 | Example5 | Example6 | Example7 | Example8 | Example9 | Comparative Example3 |
|---|---|---|---|---|---|---|---|---|
| Presence of isocyanate | | None | None | None | None | None | None | Present |
| mol equivalent ratio | Alkenyl group in compound A | 1 | 1 | 1 | 1 | 1 | 1 | - |
| | Hydrosilyl group in compound B | 10 | 10 | 10 | 10 | 10 | 10 | - |
| | OH group in compound D | 7 | 12 | 12 | 12 | 9 | 12 | - |
| | Alkenyl group in compound D | - | - | - | - | 3 | - | - |
| Water absorption (wt%) | | 60% | 500% | 450% | 400% | 930% | 640% | 1060% |
| Volume expansion coefficient (vol%) | | 3% | 3% | 10% | 2% | 4% | 10% | 90% |
| Skin irritation | Itchy feeling | ○ | ○ | ○ | ○ | ○ | ○ | × |
| | Steam feeling | ○ | ○ | ○ | ○ | ○ | ○ | Δ |
| Healing experiment (21 days later) | | Δ | ○ | ○ | ○ | ○ | ○ | ○ |
| Wound part adhesion | | ○ | ○ | ○ | ○ | ○ | ○ | × |

The above results clarify that the present invention provides a foam for medical use containing no isocyanate group and having favorable skin compatibility and a foam for medical use having a high water absorbing property and, by adjusting the components therein, a low water absorption expansion ratio which can be preferably used as a wound dressing material and the like.
The measurement and evaluation in the above examples and comparative examples were performed by the following processes under the following conditions.

(1) Oxyethylene unit content in foam resin

The weight ratio of the added polyethylene glycol relative to the foam weight or the weight ratio excluding an allyl group content in polyethylene glycol monoallyl ether was calculated to use as a content.

(2) Density

The density of the obtained foam was measured according to JISK6400. A sample was cut out into a cube measuring about 20 mm on a side, and the surface skin portion was removed for use.

(3) Open cell coefficient

The open cell coefficient of the obtained foam was measured according to ASTM D2856 (1998). A sample was cut out into a cube measuring about 20 mm on a side, and the surface skin portion was removed for use.

(4) Water absorbing property

The water absorption represented by equation (1) was determined by weighing the weight of the obtained foam before and after immersing in a physiological sodium chloride solution at 37˚C for 24 hours.

$$\text{Water absorption} = 100 \times (\text{Foam weight after immersion} - \text{Foam weight before immersion}) / (\text{Foam weight before immersion}) \quad \text{Equation (1)}$$

A sample was cut out into a cube measuring about 20 mm on a side, and the surface skin portion was removed for use.
Similarly, the volume before and after 24-hour immersion was calculated from the outer shape using a vernier caliper or a thickness gauge to determine the water absorption expansion ratio represented by equation (2).

$$\text{Water absorption expansion ratio} = 100 \times (\text{Foam volume after immersion} - \text{Foam volume before immersion}) / (\text{Foam volume before immersion}) \ (2).$$

(5) Hardness

A cylinder 15 mm in diameter was pushed into the obtained foam at a rate of 50 mm/minute to reach 25% of the sample thickness, and then stopped while remaining the state. The compressive stress after a 30-second hold was measured using a rheometer (RT-200 J-CW, manufactured by FUDOH). A sample was cut out into a cube measuring about 25 mm on a side, and the surface skin portion was removed for use.

(6) Skin irritation

A foam sheet cut into 20 mm × 20 mm was wound around the upper arm part of five volunteers with a bandage (which may be a usually commercially available bandage, such as FC non-stretchable bandage for M arm, manufactured by Hakujuji Co., Ltd.), and fixed. After 6 hours passed, relative evaluation of a steamed state of the skin was carried out based on the swollen skin and sensory evaluation of itchy feeling due to tensile stress of the foam sheet against stretching

of the skin was carried out.

Steam feeling

○: Skin was less swollen or swollen skin cannot be observed. Δ: Skin was swollen at many parts or swollen skin is observed. Itchy feeling
○: Uncomfortable feeling is hardly felt.
Δ: Uncomfortable feeling is felt.
×: Strong uncomfortable feeling, such as twitching, is felt.

(Healing experiment)

To male 9-week-old db/db mice, a full-thickness defect was formed with a ϕ6 mm biopsy punch. Then, a foam sheet was applied thereto using a surgical tape (which may be a usually commercially available surgical tape, such as FC paper tape, manufactured by Hakujuji Co., Ltd.). The foam sheet was exchanged every three days, and the healing state was observed for 21 days.
○: Formation of granulation-tissue epidermis is observed.
×: Formation of a scab and shrinkage of the wound are observed.

When the sheet was exchanged, relative evaluation of the adhesion of the foam sheet to the wound part was carried out.
○: The sheet separates without resistance.
Δ: The sheet adheres to the wound part, and resistance is felt when removing.
× The foam is torn while the foam being adhered to the wound part or the wound is disrupted due to tension.

## Claims

1. A resin foam for medical use, comprising resin containing a siloxane unit and an oxyalkylene unit in its molecular structure and containing no isocyanate derived unit.

2. The resin foam for medical use according to claim 1, wherein a water absorption represented by equation (1) when immersed in a physiological sodium chloride solution at 37°C for 24 hours is 200 wt% or more and lower than 2000 wt%:

$$\text{Water absorption} = 100 \times (\text{Foam weight after immersion} - \text{Foam weight before immersion}) / (\text{Foam weight before immersion}) \quad (1).$$

3. A flexible water absorbing resin foam, comprising resin containing a siloxane unit and an oxyalkylene unit in its molecular structure and containing no isocyanate derived unit; and having a water absorption represented by equation (1) when immersed in a physiological sodium chloride solution at 37°C for 24 hours of 200 wt% or more and lower than 2000 wt%:

$$\text{Water absorption} = 100 \times (\text{Foam weight after immersion} - \text{Foam weight before immersion}) / (\text{Foam weight before immersion}) \quad (1).$$

4. The resin foam according to claims 1 to 3, wherein the resin comprises an oxyethylene unit as at least one kind of

the oxyalkylene unit.

**5.** The resin foam according to claims 1 to 4, wherein the resin comprises the oxyethylene unit in a proportion of 5 wt% or more and lower than 80 wt%.

**6.** The resin foam according to claims 1 to 5, wherein the resin is obtained by curing a mixture containing:

an alkenyl group-containing compound (A);
a hydrosilyl group-containing compound (B); and
a hydrosilylation catalyst (C).

**7.** The resin foam according to claim 6, wherein the alkenyl group-containing compound (A) is an organic compound containing no siloxane unit in its molecular structure.

**8.** The resin foam according to claim 6 or 7, wherein the alkenyl group-containing compound (A) comprises 50 wt% or more of a polyoxyalkylene polymer containing at least one alkenyl group at a terminal.

**9.** The resin foam according to any one of claims 6 to 8, which is obtained by further blending a foaming agent (D) in the resin, and then foaming the mixture simultaneously with curing.

**10.** The resin foam according to claim 9, wherein at least one kind of the foaming agent (D) is an active hydrogen group-containing compound.

**11.** The resin foam according to claim 10, wherein the active hydrogen group-containing compound is a compound having an OH group.

**12.** The resin foam according to claim 11, wherein the compound having an OH group is at least one member selected from the group consisting of water, alcohol, and polyether polyol.

**13.** The resin foam according to claims 11 and 12, wherein at least one kind of the compound having an OH group is polyethylene glycol.

**14.** The resin foam according to any one of claims 6 to 13, wherein the amount of a hydrosilyl group in the compound (B) is 2 mol equivalent or more relative to an alkenyl group in the compound (A).

**15.** The resin foam according to any one of claims 1 to 14, wherein the density is 10 kg/m$^3$ or more and lower than 500 kg/m$^3$ and the open cell coefficient is 80% or more.

**16.** The resin foam according to any one of claims 1 to 15, comprising a foam having a density of 10 kg/m$^3$ or more and lower than 500 kg/m$^3$, a thickness of 1 mm or more and lower than 100 mm, and an open cell coefficient of 80% or more.

**17.** The resin foam according to any one of claims 1 to 16, which is obtained by uniting at least one member selected from the group consisting of a high water-absorbing resin and particles and fibers using the same.

**18.** The resin foam according to any one of claims 1 to 17, wherein a water absorption expansion ratio represented by equation (2) when immersed in a physiological sodium chloride solution at 37°C for 24 hours is lower than 50 vol%:

Water absorption expansion ratio = 100 × (Foam volume after immersion - Foam volume before immersion) / (Foam volume before immersion) (2).

immersion) (2).

**19.** A process for producing the resin foam according to any one of claims 1 to 18, comprising:

mixing a resin composition obtained by adding an alkenyl group-containing compound (A), a hydrosilylation catalyst (C), and a foaming agent (D), and, depending on the case, another additive; and

adding and mixing a hydrosilyl group-containing compound (B) for injection foaming or spray foaming.

**INTERNATIONAL SEARCH REPORT**

International application No.
PCT/JP2007/067638

A. CLASSIFICATION OF SUBJECT MATTER
*A61L15/00*(2006.01)i, *A61L31/00*(2006.01)i, *C08J9/02*(2006.01)i, *C08J9/04*(2006.01)i

According to International Patent Classification (IPC) or to both national classification and IPC

B. FIELDS SEARCHED

Minimum documentation searched (classification system followed by classification symbols)
A61L15/00, A61L31/00, C08J9/02, C08J9/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 99/24500 A1 (Kaneka Corp.), 20 May, 1999 (20.05.99), Full text & EP 1029888 A1 | 1-19 |
| X | JP 10-87995 A (Kaneka Corp.), 07 April, 1998 (07.04.98), Full text (Family: none) | 1-19 |
| X | WO 96/15194 A1 (Kaneka Corp.), 23 May, 1996 (23.05.96), Full text & US 5652276 A & EP 739948 A1 & AU 3880495 A & CN 1138870 A & CA 2181038 A | 1-19 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 December, 2007 (05.12.07) | Date of mailing of the international search report<br>18 December, 2007 (18.12.07) |
| Name and mailing address of the ISA/<br>Japanese Patent Office<br><br>Facsimile No. | Authorized officer<br><br>Telephone No. |

Form PCT/ISA/210 (second sheet) (April 2007)

**INTERNATIONAL SEARCH REPORT**

International application No.
PCT/JP2007/067638

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

A technical feature common to the invention of claim 1 and the invention of claim 3 resides in the point of "a foam of a resin which has a siloxane unit and an oxyalkylene unit in its molecular structure and does not contain a unit derived from an isocyanate group". However, from the result of the international search, it is apparent that such a technical feature is not novel, therefore, the above technical feature is not a special technical feature.

Accordingly, there is no technical relationship between both inventions involving one or more of the same or corresponding special technical features.

Thus, this international application includes two or more inventions.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3541948 B
- JP 2005516735 PCT
- JP 7051139 A
- JP 9124816 A
- JP 3569919 B
- JP 5117521 A


### Non-patent literature cited in the description


- Silicone no shijotenbo-meka senryaku to oyo tenkai. CMC, 31 January 1990